# EUROPEAN PATENT APPLICATION

(11) **EP 4 817 658 A1**
(43) Date of publication of application: **07.10.2026**
(21) Application number: 24897874.4
(22) Date of filing: 16.10.2024
(51) Int. Cl.: A61M 15/00, A24F 42/20, A24F 7/00

(54) **INHALER**

(30) Priority: 27.11.2023 KR 20230166726
(71) Applicant: KT&G Corporation, Daedeok-gu Daejeon 34337 (KR)
(72) Inventor: JUNG, Yongmi, Daejeon 34128 (KR); KIM, Moonwon, Daejeon 34128 (KR); SHIN, Jun Won, Daejeon 34128 (KR)
(74) Representative: Ter Meer und Partner mbB
(86) International application number: PCT/KR2024/015644
(87) International publication number: WO 2025/116276

(57) **Abstract**

An inhaler, according to one embodiment, comprises: a housing which is capable of accommodating a capsule containing a functional material, and includes a first surface, a second surface opposite the first surface, and a side surface between the first surface and the second surface; a mouthpiece, at least a portion of which protrudes from the first surface of the housing along a longitudinal direction extending between the first surface and the second surface; a holder which is accommodated in the housing and includes a capsule accommodation space; and a needle which is coupled to the mouthpiece and protrudes toward the capsule accommodation space, wherein a tip of the needle is insertable into the capsule accommodation space.

## Description

### TECHNICAL FIELD

Methods and apparatuses consistent with embodiments relate to an inhaler.

### BACKGROUND ART

An inhaler may deliver a target material directly into the lungs of a user. The above description is information the inventor(s) acquired during the course of conceiving the present disclosure, or already possessed at the time, and is not necessarily art publicly known before the present application was filed.

### DISCLOSURE OF THE INVENTION / TECHNICAL GOALS

Embodiments provide an inhaler accommodating a replaceable capsule.

Embodiments provide an inhaler capable of implementing sufficient inhalation resistance.

Embodiments provide an inhaler capable of preventing unintended leakage of functional material powder.

Embodiments provide an inhaler capable of securing a sufficiently long airflow path without making a size of a device too large.

Embodiments provide an inhaler with improved aesthetics by preventing a lower inlet hole that leads into a capsule from being exposed outside a housing.

Embodiments provide an inhaler capable of reducing contamination of a needle.

The technical goals obtainable from the embodiments are not limited to the above-mentioned technical goals, and other unmentioned technical goals may be clearly understood from the following description by one of ordinary skill in the art to which the present disclosure pertains.

One or more embodiments may address at least the above problems and/or disadvantages and other disadvantages not described above. Also, the embodiments are not required to overcome the disadvantages described above, and an embodiment may not overcome any of the problems described above.

### TECHNICAL SOLUTIONS

According to an aspect of an embodiment, there is provided an inhaler configured to accommodate a capsule containing a functional material. The inhaler includes a housing including a first surface, a second surface opposite to the first surface, and a side surface between the first surface and the second surface, a mouthpiece, wherein at least a portion of the mouthpiece protrudes from the first surface of the housing in a longitudinal direction from the first surface to the second surface, a holder accommodated in the housing and comprising a capsule accommodation space, and a needle coupled to the mouthpiece and protruding toward the capsule accommodation space, wherein a tip of the needle is insertable into the capsule accommodation space.

According to an aspect of an embodiment, there is provided an inhaler configured to accommodate a capsule containing a functional material. The inhaler includes a mouthpiece, a holder disposed in a longitudinal direction from the mouthpiece and comprising a capsule accommodation space, and an airflow path extending from the holder to the mouthpiece, wherein the airflow path includes a first airflow path extending into the holder in the longitudinal direction, a second airflow path extending from the first airflow path to the capsule accommodation space in a width direction perpendicular to the longitudinal direction, and a third airflow path extending from the second airflow path to the mouthpiece in the longitudinal direction.

### EFFECTS OF THE INVENTION

According to embodiments, a capsule accommodated in an inhaler may be replaced.

According to embodiments, an inhaler may implement sufficient inhalation resistance.

According to embodiments, an inhaler may prevent unintended leakage of functional material powder.

According to embodiments, an inhaler may secure a sufficiently long airflow path without making a size of a device too large.

According to embodiments, aesthetics of an inhaler may be improved by preventing a lower inlet hole that leads into a capsule from being exposed outside a housing.

According to embodiments, an inhaler may reduce contamination of a needle.

The effects of the inhaler are not limited to the above-mentioned effects, and other unmentioned effects can be clearly understood from the following description by one of ordinary skill in the art to which the present disclosure pertains.

### BRIEF DESCRIPTION OF THE DRAWINGS

The above and/or other aspects will be more apparent by describing certain embodiments with reference to the accompanying drawings, in which:
FIG. 1 is a perspective view of an inhaler according to an embodiment;
FIG. 2 is a perspective view of an inhaler from a different angle, according to an embodiment;
FIG. 3 is a front view of an inhaler from which a housing is removed, according to an embodiment;
FIG. 4 is a diagram schematically illustrating an internal structure of an inhaler, of an embodiment;
FIG. 5 is a diagram illustrating a holder of an inhaler, according to an embodiment; and
FIG. 6 is a diagram illustrating an airflow path of an inhaler, according to an embodiment.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, expressions "at least one of a, b, or c" and "at least one of a, b, and c" should be construed as referring to "a," "b," "c," "a and b," "a and c," "b and c," or "a, b, and c."

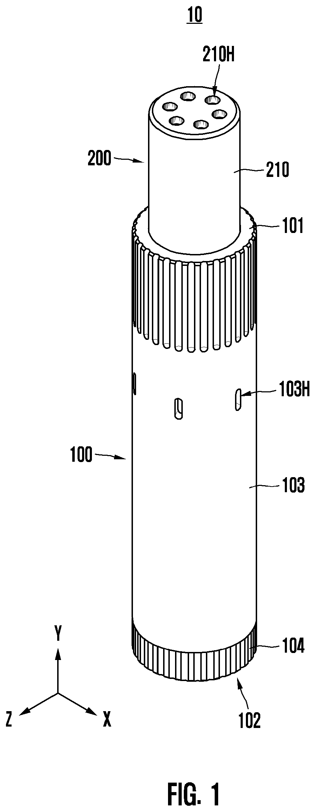

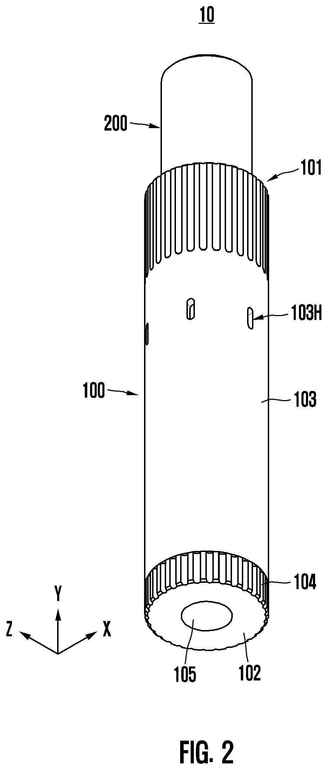

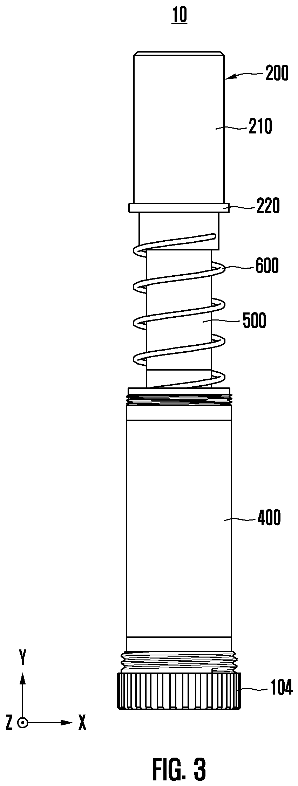

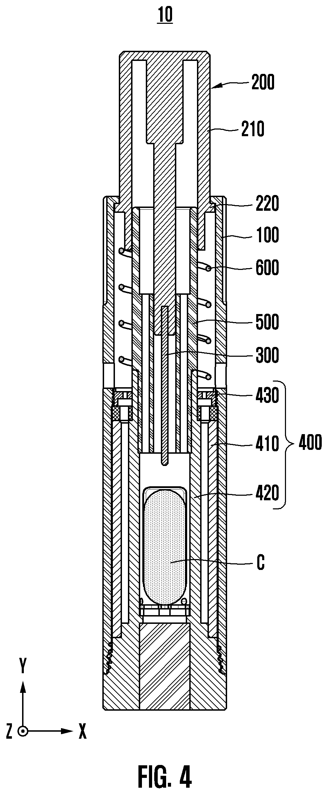

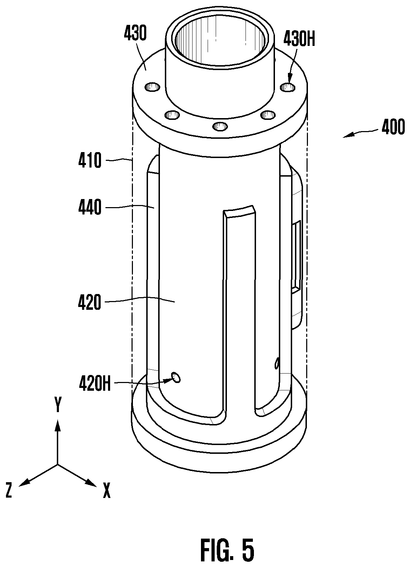

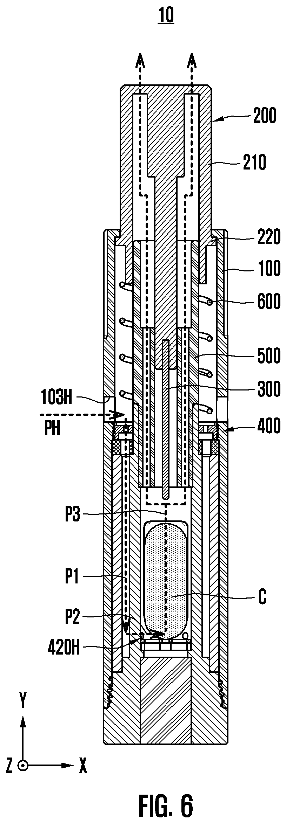

FIG. 1 is a perspective view of an inhaler 10, and FIG. 2 is a perspective view of an inhaler 10 from a different angle. FIG. 3 is a front view of an inhaler 10 from which a housing is removed, FIG. 4 is a diagram schematically illustrating an internal structure of the inhaler 10, and FIG. 5 is a diagram illustrating a holder of the inhaler 10. FIG. 6 is a diagram illustrating an airflow path of the inhaler 10.

Referring to FIGS. 1 to 4, the inhaler 10 may deliver a functional material to a user. For example, the inhaler 10 may be configured as an inhaler that delivers nicotine to the lungs of the user in a form of an aerosol.

The inhaler 10 may accommodate a capsule (e.g., a capsule C of FIG. 4), and the capsule C may contain a functional material. For example, the functional material may include at least one of nicotine, theanine, caffeine, taurine, a pharmacological substance, or a mixture thereof. The functional material may have a form of fine granules or dry powder. An outer shell of the capsule C may be composed of a material that may be penetrated or cut by a needle 300. For example, the capsule C may be filled with a functional material in an amount of 10 to 14 puffs.

The inhaler 10 may include a housing 100, a mouthpiece 200, the needle 300, and a holder 400.

The housing 100 may include a first surface 101, a second surface 102 opposite to the first surface 101, and a side surface 103 between the first surface 101 and the second surface 102. For example, the housing 100 may have an internal space defined by the first surface 101, the second surface 102, and the side surface 103. The housing 100 may be configured in a cylindrical shape, and elongated protrusions or grooves may be regularly arranged on at least a portion of the side surface 103 adjacent to the first surface 101 of the housing 100. The protrusions or grooves may provide a grip that makes it easy for the user to grip the side surface 103 of the housing 100.

The housing 100 may further include a housing cover 104 covering the second surface 102. The internal space of the housing 100 may be opened by the housing cover 104, and when the housing cover 104 is opened, the capsule C may enter or exit a capsule accommodation space. The housing cover 104 may have a circular or cylindrical shape and may include elongated protrusions or grooves regularly formed along an outer circumferential surface of the housing cover 104. The protrusions or grooves may provide a grip that makes it easy to open and close the housing cover 104.

The housing cover 104 may include a filter 105. The filter 105 may be disposed to penetrate the housing cover 104 in a longitudinal direction (e.g., a ±Y direction of FIG. 1 or FIG. 2) from the first surface 101 to the second surface 102. One end of the filter 105 may be exposed to the capsule accommodation space, and another end of the filter 105 may be exposed to external air. During an inhaling operation, external air may be introduced through the filter 105, and the external air may raise the capsule C to facilitate a discharge of functional material powder.

The filter 105 may be a cellulose acetate filter. The filter 105 may be consumables. Alternatively, the filter 105 may be used semi-permanently.

The housing 100 may include a housing inlet hole 103H through which external air is introduced into the internal space of the housing 100. The housing inlet hole 103H may be formed in the side surface 103. The housing inlet hole 103H may be a hole formed penetrating the side surface 103. For example, the housing inlet hole 103H may be configured in plurality, and the plurality of housing inlet holes 103H may be arranged at equal intervals or at equal angles along the side surface 103 of the housing 100.

At least a portion of the mouthpiece 200 may protrude from the first surface 101 of the housing 100. The mouthpiece 200 may have a mouthpiece body 210 that has a cylindrical shape. In particular, referring to FIG. 3 or FIG. 4, a mouthpiece flange may be provided on an outer surface of the mouthpiece body 210. The mouthpiece flange may protrude in a width direction (e.g., a ±X direction of FIG. 3 or FIG. 4) from the mouthpiece body 210. Based on the mouthpiece flange, an upper portion (e.g., a +Y direction side of FIG. 3 or FIG. 4) of the mouthpiece body 210 may protrude from the first surface 101 of the housing 100 to an outside of the housing 100, and based on the mouthpiece flange, a lower portion (e.g., a -Y direction side of FIG. 3 or FIG. 4) of the mouthpiece body 210 may be accommodated in the internal space of the housing 100 and may not be exposed to the outside. The mouthpiece flange may function as a stopper of the mouthpiece body 210. For example, the mouthpiece body 210 may move forward and backward in the longitudinal direction, and the mouthpiece flange may limit movement of the mouthpiece body 210 in an upward direction (e.g., the +Y direction of FIG. 3 or FIG. 4).

The mouthpiece 200 may include a mouthpiece inhalation hole 210H. The mouthpiece inhalation hole 210H may be formed at an upper end of the mouthpiece body 210 (e.g., an upper end in the +Y direction of FIG. 1) and may penetrate the mouthpiece body 210 in the longitudinal direction (e.g., the ±Y direction of FIG. 1). The mouthpiece inhalation hole 210H may be arranged to extend in a straight line with the capsule accommodation space. The functional material may be inhaled from the capsule accommodation space into the user through the mouthpiece inhalation hole 210H.

The mouthpiece inhalation hole 210H may be configured in plurality, and the plurality of mouthpiece inhalation holes 210H may be arranged in a circumferential direction of the mouthpiece body 210 at the upper end of the mouthpiece body 210. For example, referring to FIG. 1, the plurality of mouthpiece inhalation holes 210H may be regularly arranged at equal intervals or at equal angles in the circumferential direction of the mouthpiece body 210 along an edge of the upper end of the mouthpiece body 210. The plurality of mouthpiece inhalation holes 210H may prevent insufficient inhalation when inhaling the functional material and provide appropriate inhalation resistance.

In particular, referring to FIG. 4, the needle 300 may be provided at a lower end (e.g., the -Y direction side of FIG. 4) of the mouthpiece body 210. The needle 300 may be provided at a position that does not overlap the mouthpiece inhalation hole 210H at the lower end of the mouthpiece body 210. For example, the needle 300 may be coupled to a center of the lower end of the mouthpiece body 210.

For example, the needle 300 may be configured in plurality. The plurality of needles 300 may be arranged at equal intervals or at equal angles from the center of the lower end of the mouthpiece body 210. The plurality of needles 300 may have different longitudinal lengths. For example, the central needle 300 may be formed to be the longest and the peripheral needles 300 may have shorter lengths. In this case, a central portion of the capsule C may be penetrated in the longitudinal direction, and only an upper portion of an outer portion of the capsule C may be cut/crushed, so that vortex movement of airflow inside the capsule C may be smooth.

The needle 300 may move together with the mouthpiece 200. For example, when the mouthpiece 200 moves longitudinally back and forth toward the holder 400 or the capsule accommodation space within the holder 400, the needle 300 may also move longitudinally back and forth together with the mouthpiece 200. A length of the needle 300 may be set so that a tip of the needle 300 may not be inserted into the capsule accommodation space in a retracted state (e.g., a position of the needle 300 of FIG. 4). When the needle 300 is in an advanced state (e.g., when the user presses the mouthpiece 200), the tip of the needle 300 may be inserted into the capsule accommodation space, and at least a portion of the capsule C may be penetrated, cut, or crushed by the needle 300.

The needle 300 may not be exposed to the outside by the holder 400. In addition, the needle 300 may not be exposed to the outside by the housing 100. Since the needle 300 may be doubly protected by the holder 400 and the housing 100, contamination of the needle 300 due to an external environment may be reduced.

The inhaler 10 may include an elastic element 600 between the mouthpiece 200 and the holder 400. The elastic element 600 may apply an elastic force to the mouthpiece 200 to push the mouthpiece 200 back to an original position of the mouthpiece 200 when the mouthpiece 200 moves forward toward the holder 400. For example, the elastic element 600 may be configured as a coil spring.

One end of the elastic element 600 may be seated on the mouthpiece flange of the mouthpiece 200. Another end of the elastic element 600 may be seated on an upper end of the holder 400 (e.g., an upper cover 430 of the holder 400 of FIG. 4).

The mouthpiece 200 and the needle 300 may be in a retracted position (e.g., the position of the mouthpiece 200 and the needle 300 of FIG. 4) in a normal state by the elastic element 600. When the user presses the mouthpiece 200, the needle 300 may move forward together with the mouthpiece 200 to penetrate, cut, or crush the capsule C. For example, a maximum forward distance of the needle 300 may be set as a distance between the mouthpiece flange and the upper cover 430 of the holder 400. When a pressing motion of the user is released, the needle 300 may be returned to an original position of the needle 300 together with the mouthpiece 200 by the elastic element 600. In this case, the retracted position of the mouthpiece 200 may be limited by the mouthpiece flange.

Referring to FIGS. 4 and 5, the holder 400 may be positioned below the mouthpiece 200 (e.g., the -Y direction side of FIG. 4) and accommodated in the internal space of the housing 100. The holder 400 may include a first case 410 positioned on an inner side of the side surface 103, a second case 420 positioned spaced apart from the first case 410 in an inner side of the first case 410, and the upper cover 430 positioned close to the first surface 101 of the housing 100, and a capsule accommodation space may be provided in the second case 420. The first case 410 and the second case 420 may be spaced apart from each other so that a flow space through which airflow may move may be formed between the first case 410 and the second case 420. The flow space between the first case 410 and the second case 420 may be covered by an upper cover 430.

The first case 410 may have a cylindrical shape, an upper portion of the first case 410 may be covered by the upper cover 430, and a lower portion of the first case 410 may be covered by the second surface 102 of the housing 100 or by the housing cover 104.

The second case 420 may have a cylindrical shape, an upper portion of the second case 420 may be covered by the upper cover 430, and a lower portion of the second case 420 may be covered by the second surface 102 of the housing 100 or by the housing cover 104. The second case 420 may have a less diameter than the first case 410 so that the second case 420 may be accommodated in the first case 410.

The holder 400 may further include a rib 440. The rib 440 may protrude from one of the first case 410 and the second case 420 toward the other of the first case 410 and the second case 420. The rib 440 may form a separation distance between the first case 410 and the second case 420 so that the flow space may be stably formed between the first case 410 and the second case 420.

The upper cover 430 may have a donut shape. An upper portion of the flow space between the first case 410 and the second case 420 may be covered by the upper cover 430. The upper portion of the second case 420 may further extend through a center of the upper cover 430.

The upper cover 430 may include an upper cover penetration hole 430H. The upper cover penetration hole 430H may be formed to penetrate the upper cover 430 in the longitudinal direction from an upper surface of the upper cover 430. The upper cover penetration hole 430H may be configured in plurality, and the plurality of upper cover penetration holes 430H may be arranged at equal intervals or at equal angles along an outer periphery of the upper surface of the upper cover 430. By the upper case penetration hole 430H, the housing inlet hole 103H and the flow space between the first case 410 and the second case 420 may communicate with each other, and air outside the housing 100 may be introduced into the flow space.

The second case 420 may include a second case inlet hole 420H. The second case inlet hole 420H may be formed in a lower side of the second case 420 and may penetrate the second case 420 in the width direction. An inlet side of the second case inlet hole 420H may be disposed to be in contact with the flow space, and an outlet side of the second case inlet hole 420H may be disposed to be in contact with the capsule accommodation space, so that the flow space and the capsule accommodation space may communicate with each other by the second case inlet hole 420H. External air introduced into the flow space may be introduced into the capsule accommodation space through the second case inlet hole 420H.

By the first case 410, the second case inlet hole 420H may not be exposed to the outside. Thus, the inhaler 10 may secure excellent aesthetics, and the functional material powder may not be exposed to the outside of the inhaler 10.

The housing inlet hole 103H may be located closer to the first surface 101 of the housing 100 than to the upper cover 430. Accordingly, the housing inlet hole 103H may be located above the upper cover penetration hole 430H. The second case inlet hole 420H may be located at a lower portion of the upper cover penetration hole 430H.

Referring again to FIG. 3 or FIG. 4, the inhaler 10 may further include an intermediate holder 500. The intermediate holder 500 may be a tubular (or pipe-shaped) structure that may be disposed between at least a portion of the mouthpiece 200 and the holder 400. The elastic element 600 may be disposed on an outer side of the intermediate holder 500, and the needle 300 may be disposed in an inside of the intermediate holder 500. The intermediate holder 500 may serve to guide the elastic element 600 and/or the needle 300. Depending on design specifications of the inhaler 10, the intermediate holder 500 may be omitted.

The inhaler 10 may not include the housing 100. For example, the inhaler 10 may be configured not to include the housing 100 as shown in FIG. 3, in which case the elastic element 600 may be disposed to be accommodated within the intermediate holder 500 in order not to be exposed to the outside. When the inhaler 10 does not include the housing 100, external air may be introduced into the inhaler 10 through the upper cover penetration hole 430H. When the inhaler 10 does not include the housing 100, the inhaler 10 may have less volume and weight.

Referring to FIG. 6, the inhaler 10 may include an airflow path.

When the inhaler 10 does not include the housing 100, the airflow path may include a first airflow path P1 extending into the holder 400 in the longitudinal direction, a second airflow path P2 extending in the width direction from the first airflow path P1 to the capsule accommodation space, and a third airflow path P3 extending from the second airflow path P2 to the mouthpiece 200.

The first airflow path P1 may be an airflow path in which air introduced through the upper cover penetration hole 403H moves in the longitudinal direction through the flow space formed between the first case 410 and the second case 420.

The second airflow path P2 may be an airflow path penetrating the second case 420. For example, after passing through the first airflow path P1, airflow may be introduced through the second case inlet hole 420H and move from the flow space to the capsule accommodation space in the width direction.

The third airflow path P3 may be a path through which airflow flows from the capsule accommodation space to the mouthpiece inhalation hole 210H, and the third airflow path P3 may be formed in the longitudinal direction. When air moves along the third airflow path P3, functional material powder flown out of the capsule C may also move together with the air and may be inhaled by a user.

Since the airflow path of the inhaler 10 may be formed to pass through the first airflow path P1 and to connect to the second airflow path P2, the second case inlet hole 420H communicating with the capsule accommodation space may not be exposed to the outside, thereby maintaining a clean exterior. Thus, the inhaler 10 may form excellent aesthetics and may effectively prevent the functional material powder from flowing out of the inhaler 10.

Compared to a case in which the airflow path of the inhaler 10 includes only the second airflow path P2 and the third airflow path P3, the inhaler 10 may secure sufficient inhalation resistance by further including the first airflow path P1. When inhalation resistance is low, most of the functional material powder may be discharged during an initial transfer, and accordingly, uniform transfer may not be achieved. Since the airflow path of the inhaler 10 may include a first airflow path P1, sufficient inhalation resistance may be secured. Accordingly, functional material powder may be uniformly transferred while the user performs 10 to 14 puffs.

When the inhaler 10 includes the housing 100, the airflow path may further include a housing airflow path PH. The housing airflow path PH may be an airflow path that penetrates the side surface 103 of the housing 100 in the width direction.

The housing airflow path PH may be a path through which air introduced through the housing inlet hole 103H formed in the side surface 103 of the housing 100 moves to the internal space of the housing 100. After passing through the housing airflow path PH, airflow may connect to the first airflow path P1.

Since the airflow path of the inhaler 10 may include the housing airflow path PH, the first airflow path P1, the second airflow path P2, and the third airflow path P3, airflow may move in the width direction, the longitudinal direction, and the width direction, and may move in the longitudinal direction again to be delivered to the user. When the airflow path of the inhaler 10 also includes the housing airflow path PH, sufficient inhalation resistance may be secured even in a compact space.

The inhaler 10 may be implemented to include the capsule C that is easily replaceable and may secure sufficient inhalation resistance. In addition, the inhaler 10 may prevent unintended leakage of functional material powder and may secure a sufficiently long airflow path without making a size of a device too large. In addition, excellent aesthetics of the inhaler 10 may be secured by preventing a lower inlet hole (e.g., the second case inlet hole 420H) that leads to the capsule C from being exposed outside the housing 100 or the holder 400, and contamination of the needle 300 due to the external environment may be reduced by doubly protecting the needle 300 by the holder 400 and the housing 100.

The inhaler 10 may accommodate the capsule C containing a functional material, and the inhaler 10 may include the housing 100 including the first surface 101, the second surface 102 opposite to the first surface 101, and the side surface 103 between the first surface 101 and the second surface 102, a mouthpiece 200, wherein at least a portion of the mouthpiece 200 protrudes from the first surface 101 of the housing 100 in a longitudinal direction from the first surface 101 to the second surface 102, the holder 400 accommodated in the housing 100 and including the capsule accommodation space, and the needle 300 coupled to the mouthpiece 200 and protruding toward the capsule accommodation space, wherein the tip of the needle 300 may be insertable into the capsule accommodation space.

The housing 100 may include the housing inlet hole 103H through which air may be introduced into the housing 100, and the housing inlet hole 103H may be formed in the side surface 103.

The holder 400 may include the upper cover 430 disposed close to the first surface 101, the first case 410 positioned on the inner side of the side surface 103, and the second case 420 spaced apart from the first case 410 in an inner side of the first case 410, wherein the capsule accommodation space may be provided in the second case 420, the flow space may be formed between the first case 410 and the second case 420, and the flow space may be covered by the upper cover 430.

The holder 400 may further include the rib 440 protruding from at least one of the first case 410 and the second case 420 toward the other of the first case 410 and the second case 420, and the flow space may be formed by the rib 440.

The upper cover 430 may include the upper cover penetration hole 430H penetrating the upper cover 430 in the longitudinal direction, and the housing inlet hole 103H and the flow space may communicate with each other via the upper cover penetration hole 430H.

The housing inlet hole 103H may be located closer to the first surface 101 than to the upper cover 430.

The second case 420 may include the second case inlet hole 420H, and the flow space and the capsule accommodation space may communicate with each other via the second case inlet hole 420H.

The mouthpiece 200 may have a mouthpiece body 210 that has a cylindrical shape, the mouthpiece 200 may include a mouthpiece inhalation hole 210H formed in the circumferential direction of the mouthpiece body, the mouthpiece inhalation hole 210H may penetrate the mouthpiece body 210 in the longitudinal direction, and the needle 300 may be coupled to the central portion of the mouthpiece body 210.

The mouthpiece 200 and the needle 300 may be configured to move back and forth toward the holder 400.

The elastic element 600 may be provided between the mouthpiece 200 and the holder 400 so that the mouthpiece 200 may be returned to an original position by the elastic element 600.

The housing 100 may further include the housing cover 104 that covers the second surface 102, the housing cover 104 may include the filter 105, one side of the filter 105 may be exposed to the capsule accommodation space, and an other side of the filter 105 may be exposed to external air.

The inhaler 10 may further include the intermediate holder 500 disposed between the holder 400 and the mouthpiece 200.

The inhaler 10 may accommodate a capsule containing **a** functional material, and the inhaler 10 may include the mouthpiece 200, the holder 400 disposed in a longitudinal direction from the mouthpiece 200 and including the capsule accommodation space, and the airflow path extending from the holder 400 to the mouthpiece 200, wherein the airflow path may include the first airflow path P1 extending into the holder 400 in the longitudinal direction, the second airflow path P2 extending from the first airflow path P1 to the capsule accommodation space in **the** width direction perpendicular to the longitudinal direction, and the third airflow path P3 extending from the second airflow path P2 to the mouthpiece 200 in the longitudinal direction.

The inhaler 10 may further include the housing 100 including the first surface 101, the second surface 102 located opposite to the first surface 101 in the longitudinal direction, and the side surface 103 between the first surface 101 and the second surface 102, wherein at least a portion of the mouthpiece 200 may protrude from the first surface 101 of the housing 100, the airflow path may further include the housing airflow path PH formed in the side surface 103 of the housing 100 in the width direction, and the first airflow path P1 may extend from the housing airflow path PH.

The holder 400 may include the upper cover 430 disposed close to the first surface 101, the first case 410 positioned on the inner side of the side surface 103, and the second case 420 spaced apart from the first case 410 in the inner side of the first case 410, wherein the first case 410 and the second case 420 may be covered by the upper cover 430, the second case 420 may include the capsule accommodation space, a flow space may be formed between the first case 420 and the second case 420, at least a portion of the first airflow path P1 may be formed along the flow space, and at least a portion of the second airflow path P2 may be formed by passing through the second case 420.

The descriptions of the above-described embodiments are only examples, and it will be understood by one of ordinary skill in the art that various changes and equivalents may be made therefrom. Therefore, the scope of the disclosure should be defined by the appended claims, and all differences within the scope equivalent to those described in the claims will be construed as being included in the scope of protection defined by the claims.

## Claims

1. An inhaler configured to accommodate a capsule containing a functional material, the inhaler comprising:
a housing comprising:
a first surface;
a second surface opposite to the first surface; and
a side surface between the first surface and the second surface;
a mouthpiece, wherein at least a portion of the mouthpiece protrudes from the first surface of the housing in a longitudinal direction from the first surface to the second surface;
a holder accommodated in the housing and comprising a capsule accommodation space; and
a needle coupled to the mouthpiece and protruding toward the capsule accommodation space,
wherein a tip of the needle is insertable into the capsule accommodation space.

2. The inhaler of claim 1, wherein the housing comprises a housing inlet hole, wherein air is introduced into the housing through the housing inlet hole, and the housing inlet hole is formed in the side surface.

3. The inhaler of claim 2, wherein the holder comprises:
an upper cover disposed close to the first surface;
a first case positioned on an inner side of the side surface; and
a second case spaced apart from the first case in an inner side of the first case,
wherein the capsule accommodation space is provided in the second case,
wherein a flow space is formed between the first case and the second case, and
wherein the flow space is covered by the upper cover.

4. The inhaler of claim 3, wherein the holder further comprises a rib protruding from at least one of the first case and the second case toward the other of the first case and the second case, and the flow space is formed by the rib.

5. The inhaler of claim 3, wherein the upper cover comprises an upper cover penetration hole configured to penetrate the upper cover in the longitudinal direction, and the housing inlet hole and the flow space are configured to communicate with each other via the upper cover penetration hole.

6. The inhaler of claim 5, wherein the housing inlet hole is located closer to the first surface than to the upper cover.

7. The inhaler of claim 3, wherein the second case comprises a second case inlet hole, and the flow space and the capsule accommodation space are configured to communicate with each other via the second case inlet hole.

8. The inhaler of claim 1, wherein
the mouthpiece has a mouthpiece body that has a cylindrical shape,
the mouthpiece comprises a mouthpiece inhalation hole formed in a circumferential direction of the mouthpiece body,
the mouthpiece inhalation hole is configured to penetrate the mouthpiece body in the longitudinal direction, and
the needle is coupled to a central portion of the mouthpiece body.

9. The inhaler of claim 1, wherein the mouthpiece and the needle are capable of moving back and forth toward the holder.

10. The inhaler of claim 9, wherein an elastic element is provided between the mouthpiece and the holder, and the mouthpiece is configured to be returned to an original position of the mouthpiece by the elastic element.

11. The inhaler of claim 1, wherein the housing further comprises a housing cover configured to cover the second surface, and the housing cover comprises a filter, wherein one side of the filter is exposed to the capsule accommodation space, and another side of the filter is exposed to external air.

12. The inhaler of claim 1, further comprising an intermediate holder disposed between the holder and the mouthpiece.

13. An inhaler configured to accommodate a capsule containing a functional material, the inhaler comprising:
a mouthpiece;
a holder disposed in a longitudinal direction from the mouthpiece and comprising a capsule accommodation space; and
an airflow path extending from the holder to the mouthpiece,
wherein the airflow path comprises:
a first airflow path extending into the holder in the longitudinal direction;
a second airflow path extending from the first airflow path to the capsule accommodation space in a width direction perpendicular to the longitudinal direction; and
a third airflow path extending from the second airflow path to the mouthpiece in the longitudinal direction.

14. The inhaler of claim 13, further comprising:
a housing comprising:
a first surface;
a second surface located opposite to the first surface in the longitudinal direction; and
a side surface between the first surface and the second surface,
wherein at least a portion of the mouthpiece protrudes from the first surface of the housing,
wherein the airflow path further comprises a housing airflow path formed in a side surface of the housing in the width direction, and
wherein the first airflow path extends from the housing airflow path.

15. The inhaler of claim 14, wherein the holder comprises:
an upper cover disposed close to the first surface;
a first case positioned on an inner side of the side surface; and
a second case spaced apart from the first case in an inner side of the first case,
wherein
the first case and the second case are covered by the upper cover,
the second case comprises the capsule accommodation space,
a flow space is formed between the first case and the second case,
at least a portion of the first airflow path is formed along the flow space, and
at least a portion of the second airflow path is formed by passing through the second case.
